# EUROPÄISCHE PATENTSCHRIFT

(11) **EP 1 207 901 B1**
(45) Veröffentlichungstag und Bekanntmachung des Hinweises auf die Patenterteilung: **08.10.2003**
(21) Anmeldenummer: 00960578.3
(22) Anmeldetag: 01.09.2000
(51) Int. Cl.: A61K 38/22, A23L 3/3463, A23B 7/10, A23C 9/13, C12H 1/052, A61P 31/02

(54) **VERWENDUNG NATRIURETISCHER PEPTIDE ALS ANTIBIOTISCH WIRKSAME SUBSTANZEN ZUR BEHANDLUNG VON BAKTERIELLEN INFEKTIONEN**
USE OF NATRIURETIC PEPTIDES AS ANTIBIOTICALLY EFFECTIVE SUBSTANCES FOR TREATING BACTERIAL INFECTIONS
UTILISATION DE PEPTIDES NATRIURETIQUES COMME SUBSTANCES A ACTION ANTIBIOTIQUE DANS LE CADRE DU TRAITEMENT DES INFECTIONS BACTERIENNES

(30) Priorität: 03.09.1999 DE 19942230
(43) Veröffentlichungstag der Anmeldung: 29.05.2002
(73) Patentinhaber: Pharis Biotec GmbH, 30625 Hannover (DE)
(72) Erfinder: FORSSMANN, Wolf-Georg, 30175 Hannover (DE); KRAUSE, Alexander, 31737 Rintel (DE); MARONDE, Erik, 30900 Wedemark (DE)
(74) Vertreter: Meyers, Hans-Wilhelm, Dr.Dipl.-Chem.
(86) Internationale Anmeldenummer: EP0008545
(87) Internationale Veröffentlichungsnummer: WO01017548

(56) Entgegenhaltungen:
- WO-A-95/28952

## Beschreibung

Die Erfindung betrifft die Anwendung natriuretischer Peptide (ANP, BNP, CNP und Urodilatin), als antibiotisch wirksame Peptid-Präparate. Die Gewinnung erfolgt durch eine chemische Peptidsynthese oder biotechnologische Herstellung und Konfektionierung als galenisch zubereitete Substanz zur medizinischen und tiermedizinischen Verwendung als Medikament.

Bei den Peptiden, die Gegenstand dieser Erfindung sind, handelt es sich um Mitglieder der Familie der natriuretischen Peptide. Atriales natriuretisches Peptid (ANP) der Ratte (Flynn et al., 1983), des Schweins (Forssmann et al., 1983, 1984), und des Menschen (Kangawa and Matsuo, 1984) wurde in seiner Primärstruktur beschrieben. Die in der Niere auftretende Form des ANP, Urodilatin, wurde von Forssmann und Mitarbeitern zuerst 1988 isoliert (Schulz-Knappe et al., 1988). Das Homologe des atrialen natriuretischen Peptides (ANP), das Braintype natriuretische Peptid (BNP) wurde von Sudoh und Mitarbeitern 1988 erstmals isoliert. Eine antibiotische Wirksamkeit wurde für natriuretische Peptide bislang nicht vermutet. Für den Nachweis einer antimikrobiellen Aktivität wird vorzugsweise ein Test durchgeführt, der für basische Peptide geeignet ist. Hier ist der Wachstumshemmtest von Lehrer und Mitarbeitern geeignet, um eine antibiotische Aktivität zu erkennen (Lehrer et al., J. Immun Methods, Vol. 137, S. 167 1991).

Das der Erfindung zugrunde liegende technische Problem bestand in der Bereitstellung antibiotisch wirksamer Mittel. Gelöst wird das Problem durch die Verwendung von natriuretischen Peptiden (Natriubiotika ausgewählt aus der Gruppe ANP, BNP, CNP und Urodilatin).

Erfindungsgemäß werden die Natriubiotika verwendet zur Herstellung eines antibiotisch wirksamen Mittels zur Behandlung einer pathogen veränderten bakteriellen Flora im Magendarmtrakt, respiratorischem und urogenitalem System, der Haut sowie der Verwendung in der Lebensmitteltechnologie als Hilfsstoff bei Gärprozessen und als Konservierungsstoff.

Bei der Verwendung als Arzneimittel werden die Natriubiotika vorzugsweise in Mengen von 1 µg - 1 mg pro Einheit in für Infusionen geeigneten Medien, Salben, Tabletten, Sprays, "slow release"-Kapseln formuliert.

Die Verwendung der Natriubiotika umfasst auch die Behandlung von Veränderungen der Darmflora, die Behandlung mikrobiell ausgelöster Hauterkrankungen, Behandlung von Abberationen der humanen Vaginalflora. Die erfindungsgemäße Verwendung von Natriubiotika in der Lebensmitteltechnologie umfasst auch die Verwendung als Konservierungsstoff für Lebensmittel oder andere verderbliche Waren, als Hilfsstoff bei industriellen Gärprozessen, z. B. bei der Bierherstellung, bei der Joghurtproduktion sowie der Sauerkrautherstellung.

Humanes ANP 99-126 und Urodilatin wirken überraschenderweise auf grampositive Bakterien wie B.subtilis, M. luteus und S. carnosus, und auf gramnegative Bakterien wie E. coli, N. cinerea und P. fluorescens sowie die Hefe S. cerevisiae wachstumshemmend. Ebenso wirkt humanes BNP-312 in der gleichen Art auf grampositive Bakterien wie B.subtilis, M. luteus und S. carnosus und gramnegative Bakterien wie E. coli, N. cinerea und P. fluorescens sowie die Hefe S. cerevisiae, wachstumshemmend. Die wachstumsmodulierende Eigenschaft dieser spezifischen natriuretischen Peptide auf bestimmte Keime wurde erstmals eindeutig nachgewiesen.

Über die chemische und biotechnologische Synthese können die natriuretischen Peptide in hochreiner und biologisch aktiver Form hergestellt, und als Medikament eingesetzt werden.

Die erfindungsgemäß verwendbaren Stoffe, bestehend aus den synthetischen und rekombinanten Produkten, können die bakterielle Flora des Darms, der Haut und anderer bakteriell besiedelter Körperzonen verändern und bei einer bakteriellen Fehlbesiedlung zu einer Verbesserung der Keimflora führen. Die dargestellten Reinstoffe lassen sich daher zur Bekämpfung von Durchfällen, insbesondere Säuglingsdurchfällen, also Infektionen des Magendarmtraktes, zusätzlich aber auch des Respirationssystems, des Urogenitalapparates und bei Hautinfektionen, verwenden. Die Präparate sind als Zusatzstoffe für Lebensmittel oder als Therapeutika verwendbar und dienen bei der Herstellung von Lebensmitteln als Hilfsstoffe, insbesondere bei Lebensmitteln, die durch Gärung und andere bakterielle Prozesse hergestellt werden. Bei diesen Präparaten handelt es sich um natürliche Konservierungsmittel.

Die Erfindung wird nachfolgend anhand von Beispielen und den folgenden Figuren, auf die in den Beispielen Bezug genommen wird, erläutert:

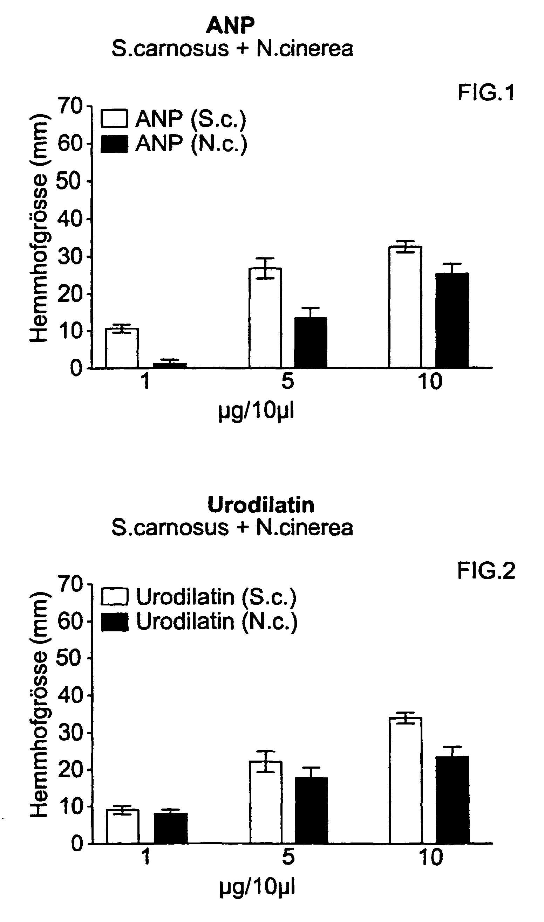

Es zeigen
Fig. 1 Wachstumshemmtest von ANP
   Radialer Diffusions-Wachstumshemmtest mit Streptococcus camosus und N. cinerea nach Lehrer und Mitarbeitern (Lehrer et al., J Immun Methods, Vol 137, S. 167 1991). Der Wachstumshemmtest ist für den Nachweis antibiotischer Peptide besonders geeignet, da als Trägermaterial statt des sonst üblichen Agar-Agars eine spezielle Agarose verwendet wurde, die keine fixierten Ladungszentren enthält. Nachweisbar sind Hemmhöfe nach Auftrag von 1 µg ANP (beide Keime).
Fig. 2 Wachstumshemmtest von Urodilatin
   Radialer Diffusions-Wachstumshemmtest mit Streptococcus carnosus und N. cinerea nach Lehrer und Mitarbeitern (Lehrer et al., J Immun Methods, Vol 137, S. 167 1991). Der Wachstumshemmtest ist für den Nachweis antibiotischer Peptide besonders geeignet, da als Trägermaterial statt des sonst üblichen Agar-Agars eine spezielle Agarose verwendet wurde, die keine fixierten Ladungszentren enthält. Nachweisbar sind Hemmhöfe nach Auftrag von 1 µg ANP (beide Keime).

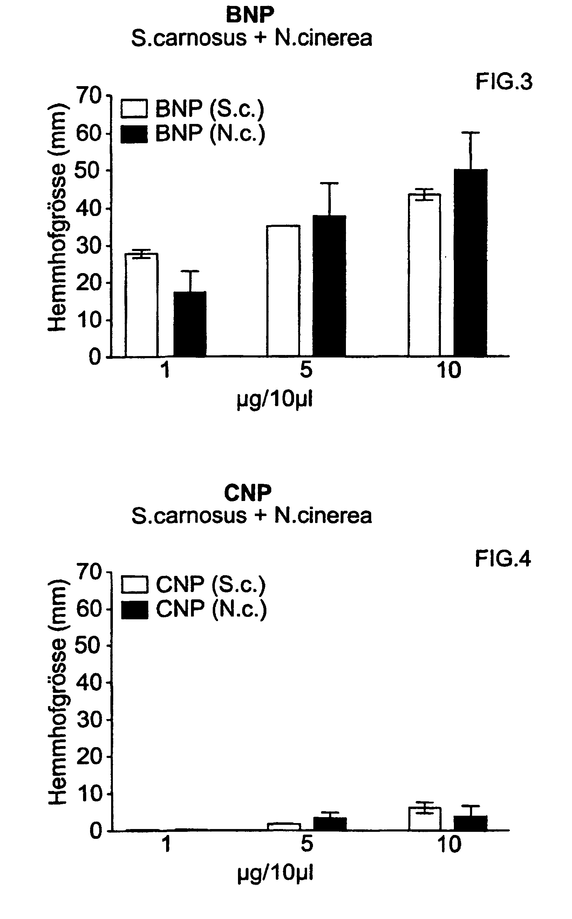

Fig. 3 Wachstumshemmtest von BNP-32
   Radialer Diffusions-Wachstumshemmtest mit Streptococcus carnosus und N. cinerea nach Lehrer und Mitarbeitern (Lehrer et al., J Immun Methods, Vol 137, S. 167 1991). Der Wachstumshemmtest ist für den Nachweis antibiotischer Peptide besonders geeignet, da als Trägermaterial statt des sonst üblichen Agar-Agars eine spezielle Agarose verwendet wurde, die keine fixierten Ladungszentren enthält. Nachweisbar sind Hemmhöfe nach Auftrag von 0,1 µg BNP (beide Keime).
Fig. 4 Wachstumshemmtest von CNP
   Radialer Diffusions-Wachstumshemmtest mit Streptococcus carnosus und N. cinera nach Lehrer und Mitarbeitern (Lehrer et al., J Immun Methods, Vol 137, S. 167 1991). Der Wachstumshemmtest ist für den Nachweis antibiotischer Peptide besonders geeignet, da als Trägermaterial statt des sonst üblichen Agar-Agars eine spezielle Agarose verwendet wurde, die keine filierten Ladungszentren enthält. Nachweisbar sind Hemmhöfe nach Auftrag von 7 µg (S.carnosus) und 11 µg (N. cinera) CNP.

### Beispiel 1:

### Chemische Synthese der antibiotisch aktiven Peptide ANP, BNP, Urodilatin und CNP

### Strategie der Synthese humaner natriuretischer Peptide:

Für die Synthese der Peptide mit den Sequenzen: 
Ser-Leu-Arg-Arg-Ser-Ser-Cys-Phe-Gly-Gly-Arg-Met-Asp-Arg-Ile-Gly-
Ala-Gln-Ser-Gly-Leu-Gly-
 Cys-Asn-Ser-Phe-Arg-Tyr (ANP/CDD) Ser-Phe-Lys-Met-Val-Gln-Gly-Ser-Gly-Cys-Phe-Gly-Arg-Lys-Met-Asp-Arg- Ile-Ser-Ser-SerSer-Gly-Leu-Gly-Cys-Lys-Val-Leu-Arg-Arg-His (BNP) 
Gly-Leu-Ser-Lys-Gly-Cys-Phe-Gly-Leu-Lys-Leu-Asp-Arg-Ile-Gly-Ser-Met-
Ser-Gly-LeuGly-Cys (CNP)
 
Thr-Ala-Pro-Arg-Ser-Leu-Arg-Arg-Ser-Ser-Cys-Phe-Gly-Gly-Arg-Met-Asp-
Arg-Ile-Gly-Ala-Gln-
 Ser-Gly-Leu-Gly-Cys-Asn-Ser-Phe-Arg-Tyr (Urodilatin) wird die Durchflussmethode (Atherton und Sheppard, in "Solid Phase Peptide Synthesis", IRL-Press, Oxford 1989) angewendet. Die genannte Peptidsequenz wird mit Hilfe einer automatischen Peptid-Syntheseapparatur (Minigen 9050) unter Verwendung von Fmoc-Aminosäuren synthetisiert. Die Fmoc-Aminosäuren waren L-konfiguriert und in vierfachem Überschuss eingesetzt.

Folgende Aminosäurederviate wurden für die Synthese verwendet:
Fmoc-Lys (Boc), Fmoc-Arg (Pmc), Fmoc-His (Trt), Fmoc-Glu (OtBu), Fmoc-Ser(tBu), Fmoc-Gln(Trt), Fmoc-Leu, Fmoc-Phe, Fmoc-Ile, Fmoc-Val. Es fehlen Cys, Gly, Met, Asp

Die Synthese wird mit trägergebundener C-terminaler Aminosäure (0,091 mmol Alanin/g Harz) an Fmoc-L-Ala-PEG-PS (Millipore) durchgeführt. Alle Kopplungen von Aminosäurederivaten werden in Anwesenheit von O-(1H-Benzotriazol--1-yl)-N,N,N',N'tetramethyluronium-tetrafluorborat (TBTU), 1-Hydroxybenzotriazol, Diisopropylethylamin durchgeführt. Folgende Synthesezyklen werden verwendet:
- Fmoc-Abspaltung mit 20% Piperidin in DMF für 10 min
- Waschen mit DMF für 12 min
- Acylierung für 30 min
- Waschen mit DMF für 8 min

Die Synthese wird durch kontinuierliche UV-Detektion verfolgt. Die Synthese wird mit der Abspaltung des N-terminalen Fmoc-Restes abgeschlossen. Das harzgebundene Peptid wird dreimal mit je 50 ml Isopropanol, Eisessig, Isopropanol und Diethylether gewaschen und getrocknet.

Die Peptide werden vom Trägerharz durch Zugabe einer Mischung von TFA-Ethandithiol/Wasser 94:3:3 (v/v/v) abgespalten und mit Ether gefällt.

Die Reinigung des Peptides erfolgt mittels Reversed-Phase-HPLC mit einer C18-Säule (Vydac, 10 pmm 300 A, 20 x 250 mm, Detektion bei 230 nm). Als Laufmittel wurden verwendet: Eluent A: 0,06 % Trifluoressigsäure (TFA), Eluent B: 0,06% TFA in Acetonitril/Wasser (4:1). Die Flussrate ist 10 ml/min, der Gradient ist folgendermaßen: von 20 % B bis 80 % B innerhalb von 70 min. Die reinen Fraktionen werden gepoolt und lyophilisiert.

Die Reinheit und Identität der Peptide wird mit Hilfe der Massenspektrometrie (QuadrupolElektrospraymassenspektrometrie, Sciex API 111, Perkin Elmer) und Sequenzierung in einem Gasphasensequenator (Model 470, Applied Biosystems, Weiterstadt) durchgeführt sowie mit Kapillarzonenelektrophorese überprüft. Die biologische Aktivität wird durch den Wachstumshemmtest kontrolliert.

### Beispiel 2:

### Rekombinante Herstellung

Die rekombinante Herstellung erfolgt nach üblichen Methoden und führt zu gleichartig reinem Peptid zur galenischen Verwendung.

## Patentansprüche

1. Verwendung der Natriubiotika ausgewählt aus der Gruppe bestehend aus ANP, BNP, CNP, Urodilatin zur Herstellung eines antibiotisch wirksamen Mittels zur Behandlung einer pathogen veränderten bakteriellen Flora in Magendarmtrakt, respiratorischem und urogenitalem System sowie der Haut.

2. Verwendung nach Anspruch 1, wobei die Natriubiotika in Mengen von 1 µg-1 mg pro Einheit, zubereitet für Infusionen, Salben, Tabletten, Sprays, "slow release"-Kapseln.

3. Verwendung gemäß Anspruch 1 und/oder 2 zur Behandlung von Veränderungen der Darmflora.

4. Verwendung gemäß Anspruch 1 und/oder 2 zur Behandlung von mikrobiell ausgelösten Hauterkrankungen.

5. Verwendung gemäß Anspruch 1 und/oder 2 zur Behandlung von Abberationen der humanen Vaginalflora.

6. Verwendung der Natriubiotika ausgewählt aus der Gruppe bestehend aus ANP, BNP, CNP, Urodilatin in der Lebensmitteltechnologie als Hilfsstoff bei Gärprozessen und als Konservierungsstoff.

7. Verwendung gemäß Anspruch 6 als Konservierungsstoff von Lebensmitteln oder anderen verderblichen Waren.

8. Verwendung gemäß Anspruch 6 als Hilfsstoff bei industriellen Gärprozessen, z.B. bei der Bierherstellung, bei der Joghurtproduktion und bei der Sauerkrautherstellung.

## Claims

1. Use of natriubiotics selected from the group consisting of ANP, BNP, CNP, urodilatin for the preparation of an antibiotically active agent for the treatment of a pathogenically altered bacterial flora in the gastro-intestinal tract, respiratory and urogenital systems and the skin.

2. The use according to claim 1, wherein said natriubiotics are formulated in amounts of from 1 µg to 1 mg per unit for infusions, ointments, tablets, sprays or sustained release capsules.

3. The use according to claims 1 and/or 2 for the treatment of alterations of the intestinal flora.

4. The use according to claims 1 and/or 2 for the treatment of microbially induced skin diseases.

5. The use according to claims 1 and/or 2 for the treatment of aberrations of the human vaginal flora.

6. Use of natriubiotics selected from the group consisting of ANP, BNP, CNP, urodilatin in food technology as an auxiliary agent in fermenting processes and as a preservative.

7. The use according to claim 6 as a preservative for foods or other perishable goods.

8. The use according to claim 6 as an auxiliary agent in industrial fermenting processes, e.g., in beer production, in yogurt production and in sauerkraut production.

## Revendications

1. Utilisation des substances natriurétiques choisies dans le groupe formé par l'ANP, le BNP, le CNP, et l'urodilatine pour préparer des agents actifs antibiotiques destinés au traitement d'une flore bactérienne modifiée par des germes pathogènes dans le système gastro-intestinal, ou dans le système respiratoire et le système urogénital et de la peau.

2. Utilisation selon la revendication 1, dans laquelle les substances natriurétiques sont préparées en des quantités de 1 µg à 1 mg par unité sous forme d'infusions, de pommades, de comprimés, de sprays ou de gélules à libération lente.

3. Utilisation selon la revendication 1 et/ou 2 pour le traitement des modifications de la flore intestinale.

4. Utilisation selon la revendication 1 et/ou 2 pour le traitement des dermatoses d 'origine microbienne.

5. Utilisation selon la revendication 1 et/ou 2 pour le traitement des aberrations de la flore vaginale humaine.

6. Utilisation des substances natriurétiques choisies parmi l'ANP, le BNP, le CNP, et l'urodilatine dans l'industrie alimentaire en tant qu'adjuvant dans les procédés de fermentation et en tant que conservateur.

7. Utilisation selon la revendication 6 en tant que conservateur des aliments ou d'autres marchandises périssables.

8. Utilisation selon la revendication 6 en tant qu'adjuvant dans les procédés industriels de fermentation, par ex. dans la fabrication de la bière, la production de yaourts et la préparation de la choucroute.
